# EUROPEAN PATENT APPLICATION

(11) **EP 2 484 693 A1**
(43) Date of publication of application: **08.08.2012**
(21) Application number: 10820253.2
(22) Date of filing: 16.08.2010
(51) Int. Cl.: C07K 16/18, A61K 39/395, A61P 35/00, A61P 35/04, C07K 16/46, G01N 33/53, C12N 15/09, C12P 21/08

(54) **HUMAN SERUM AMYLOID-A3 ANTIBODY AND USE THEREOF**

(30) Priority: 02.10.2009 JP 2009230361
(71) Applicant: Tokyo Women's Medical University, Tokyo 162-8666 (JP)
(72) Inventor: MARU, Yoshiro, Tokyo 108-0071 (JP); TANAKA, Takeshi, Tokyo 158-0097 (JP); TERAI, Kiyomi, Kuki-shi Saitama 340-0203 (JP)
(74) Representative: Hartz, Nikolai
(86) International application number: PCT/JP2010/063813
(87) International publication number: WO 2011/040133

(57) **Abstract**

The objective of the invention is to prepare an antibody to human SAA3 whose in vivo presence has not yet been identified in humans, thereby revealing the presence of in vivo human SAA3 and the relationship between human SAA3 and disease, and also to provide a method for treating such disease. More specifically our objective is to create an antibody to counter human serum amyloid-A3 and a kit for detecting and measuring human serum amyloid-A3 employing the aforementioned antibody, as well as a therapeutic cancer agent that can be utilized in methods to treat and diagnose cancer.

## Description

### Technical Field

The present invention relates to a human serum amyloid-A3 antibody and use of the same, more specifically to a human serum amyloid-A3, a method for producing the aforementioned antibody, a method for detecting and measuring human serum amyloid-A3, a method for treating and diagnosing cancer and the like that utilize the aforementioned antibody.

### Background Art

Serum amyloid A (SAA) is known as a blood precursor of amyloid A protein, which is deposited in tissue in secondary amyloidosis.

SAA is known to have 4 isotypes in mice, rabbits, horses and the like including SAA1, SAA2, SAA3 and SAA4.

Among these SAAs, SAA1 and SAA2 are acute-phase reactants which respond to inflammatory cytokines such as interleukin (IL)-1, IL-6 and tumor necrosis factor (TNF), while SAA4 is a high density lipoprotein (HDL) constituent protein.

On the other hand, it is known that SAA3 is recognized by Toll-like receptor 4 (TLR4) to activate an immune response signal via nuclear factor kB (NFkB), thereby promoting cancer metastasis. It has also been confirmed that when an anti-SAA3 antibody is given to a cancer-carrying mouse with subcutaneously transplanted cancer cells, this series of signal pathways (S100A8-SAA3-TLR4) is blocked, thereby inhibiting metastasis. TLR4 acts as a natural immune system receptor in recognizing a toxin such as a lipopolysaccharide (LPS) and thus is responsible for preventing infection. This suggests that an inflammation-like environment established by the natural immune system whose primary responsibility is to protect against infection actually serves to promote cancer cell migration (Non-Patent Document 1). Thus, in mice, SAA3 is implicated in cancer metastasis.

Human SAA3 is assumed to consist of the amino acid sequence represented by SEQ ID NO:1 based on homology with the amino acid sequence of mouse SAA3 and the like (Patent Document 1). Nevertheless, the presence of human SAA3 has been confirmed only at the level of mRNA, and no human in vivo protein expression has actually been identified.

### Citation List

### [Patent Document]

[Patent Document 1] Unexamined Japanese Patent Application Publication No. 2006-516194
[Non Patent Document]

[Non-Patent Document 1] Sachie Hiratsuka, Akira Watanabe, Yoshiko Sakurai, Sachiko Akashi-Takamura, Sachie Ishibashi, Kensuke Miyake, Masabumi Shibuya, Shizuo Akira, Hiroyuki Aburatani and Yoshiro Maru. Nature Cell Bioll10 1349-1355 (2008).

### Summary of the Invention

### Problems that the Invention is to solve

The object of the present invention is to prepare an antibody to human SAA3 whose in vivo presence has not yet been identified in human, thereby revealing the presence of in vivo human SAA3 and the relationship between human SAA3 and disease, as well as to provide a method for treating such disease.

### Means for solving the Problem

Based on the findings described above and according to standard methodology, the inventors of the present invention attempted to encourage E.coli to express a recombinant human SAA3 protein which could then be used as an antigen to produce antibodies to detect human SAA3 in a human specimen thereby revealing the presence of the human SAA3. However, the recombinant human SAA3 protein was not employed successfully as an antigen for immunization because of its extremely low expression level. The inventors also used standard methodology for producing antibodies in attempt to combine a conjugate of a peptide consisting of an amino acid sequence characteristic to human SAA3 (positions 49 to 60 of the SAA3 amino acid sequence represented by SEQ ID NO:1) with a biopolymer to create an antigen. However, the antibody titer exhibited almost no increase several months after immunization, indicating no satisfactory production of antibodies.

However, the inventors subsequently discovered that when using as an antigen a peptide conjugate comprising a partial amino acid sequence of human SAA3 which is slightly longer than the amino acid sequence characteristic to human SAA3 combined with a biopolymer, the immunized animal exhibits an increased ability to produce antibodies, thus obtaining antibodies to human SAA3 for the first time.

This human SAA3 antibody was then employed to conduct western blot analysis of specimens from normal and cancer subjects, in which the human SAA3 antibody reaction product was detected only in samples from the cancer subjects. Also, since the position of this reaction product was similar to that of the antibody reaction product of the human SAA3 recombinant protein as discussed above, the in vivo presence of human SAA3 was established in humans.

Based on this, it was discovered that human SAA3 does exist in vivo in human and can be detected using a human SAA3 antibody, and also that human SAA3 is related to cancer, thus establishing the present invention.

The present invention is as follows:
(1) An human serum amyloid-A3 antibody to counter human serum amyloid-A3.
(2) A method for producing a human serum amyloid-A3 antibody comprising:
   binding a peptide comprising positions 48 to 60 of the amino acid sequence of a human serum amyloid-A3 represented by SEQ ID N0:1 as a partial amino acid sequence with a biopolymeric compound to form a conjugate; and
   immunizing an animal with the conjugate and obtaining antibodies from the animal.
(3) A method for detecting and measuring a human serum amyloid-A3 comprising allowing the human serum amyloid-A3 antibody to act on a human specimen to detect and measure a human serum amyloid-A3 in the specimen.
(4) A method for diagnosing a cancer comprising allowing the human serum amyloid-A3 antibody to act on a human specimen to detect and measure a human serum amyloid-A3 in the specimen.
(5) A detection and measurement kit for a human serum amyloid-A3 comprising use of a human serum amyloid-A3 antibody.
(6) A therapeutic cancer agent comprising a human serum amyloid-A3 antibody as an active ingredient.
(7) A method for treating cancer comprising administering a human serum amyloid-A3 antibody to a cancer patient and allowing the antibody to bind with in vivo human serum amyloid-A3.

### Advantage of the Invention

Since the human SAA3 antibody of the invention binds to human SAA3 whose expression was not previously verified in human, it can be utilized to study the role human SAA3 plays in vivo in the human body.

In addition, since mouse SAA3 is implicated in cancer metastasis, the human SAA3 antibody of the invention can be utilized in the treatment of cancer by inhibiting metastasis, as well as in diagnosis.

### Brief Description of the Drawings

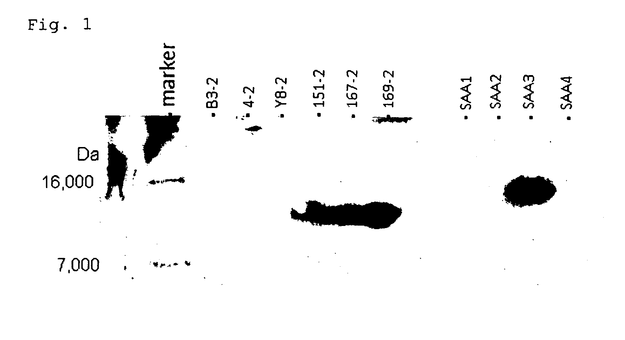

Figure 1 indicates the presence of human SAA3 in the serum of a cancer patient in a western blot analysis employing the human SAA3 monoclonal antibody (h-SAA3-3-1 antibody).

### Mode for carrying out the Invention

The invention's human SAA3 antibody is not particularly limited given that it is an antibody capable of binding to human SAA3. Also, the method for producing such an antibody is not particularly limited, and may conform to standard methodology for producing antibodies (G.Kohler and C.Milstein, Continuous cultures of fused cells secreting antibody of predefined specificity, Nature 256 495-497(1975)) except in the use of human SAA3 as an antigen.

In the present invention, a monoclonal antibody which binds specifically to human SAA3 is especially preferred among the human SAA3 antibodies discussed above. As used herein, "specific" means reacting with human SAA3 but not with human SAA1, SSA2 or SAA4, and preferably reacting exclusively with human SAA3.

Such a human SAA3 monoclonal antibody can be obtained, for example, by binding a peptide comprising positions 49 to 60 of the amino acid sequence of human SAA3 represented by SEQ ID N0:1 as a partial amino acid sequence with a biopolymeric compound to form a conjugate. An animal is immunized with this conjugate and antibodies are then obtained from the animal.

The peptide comprising positions 49 to 60 of the amino acid sequence of human SAA3 represented by SEQ ID NO:1 can, for example, be a peptide consisting of positions 19 to 60 of the amino acid sequence of human SAA3 represented by SEQ ID NO:1; more preferable is a peptide consisting of the amino acid sequence of positions 30 to 60; especially preferable is a peptide consisting of the amino acid sequence of positions 38 to 60. By using such a peptide, a human SAA3 monoclonal antibody can be obtained which recognizes a peptide consisting of positions 49 to 60 of the amino acid sequence of human serum amyloid-A3 represented by SEQ ID NO:1 or positions 38 to 48. Although such a peptide can be expressed by E.coli or can be a synthetic peptide, a synthetic peptide is preferable in view of the concern about saccharide chains and the like.

The biopolymeric compound used for binding with the peptide can be, for example, a bovine serum albumin, a keyhole limpet hemocyanine, an ovalbumin or the like.

The conjugation of the peptide with the biopolymeric compound can be accomplished by a known conjugation method such as the carbodiimide method, the maleimide method or the like.

The conjugate of the peptide and biopolymeric compound obtained as described above is dissolved in a solvent such as physiological saline and then combined and stirred with polyvinylpyrrolidone, followed by mixing with complete Freund's adjuvant to yield an adjuvant.

This adjuvant was given according to standard methodology to an animal such as a rabbit or a mouse via subcutaneous administration or similar, and was conducted once or several times at 2 to 3 week intervals for an immunization period of several months. When the antibody titer of the animal reached about 2^{5∼10} after immunization, immunization was terminated.

When the animal has completed immunization as described above, blood is taken from it using standard methodology. The serum is then separated and the antibody fraction purified, thus yielding human SAA3 antibodies.

Also according to standard methodology, polyethylene glycol is used to fuse myeloma cells with splenocytes obtained by immunizing a mouse with the conjugate to form a hybridoma, and this hybridoma is screened to obtain human SAA3 monoclonal antibodies.

This hybridoma can be screened according to standard protocols, for example, by using a peptide consisting of human SAA3 or a partial peptide thereof. The partial peptide employed in the screening can be a sequence specific to human SAA3 (positions 49 to 60 of the amino acid sequence of human SAA3 represented by SEQ ID NO:1).

The hybridoma which produces the human SAA3 monoclonal antibody that especially recognizes a peptide consisting of positions 49 to 60 of the amino acid sequence of the human SAA3 represented by SEQ ID NO:1 among human SAA3 monoclonal antibodies obtained as described above (hereinafter referred to as h-SAA3-3-1 antibody) was designated as h-SAA3-3-1, and was deposited on 28 August 2009 (FERM BP-11176) at the International Patent Organism Depositary, National Institute of Advanced Industrial Science and Technology, Tsukuba 305-8566, Japan (Higashi 1-Chome, Tsukuba-shi, Ibaraki-ken, 305-8566, Japan).

The human SAA3 antibody of the invention thus obtained binds to human SAA3. As a result, by allowing the invention's human SAA3 antibody to act on a human specimen such as human blood, serum, urine, tissue or the like, human SAA3 can be detected and measured in the human specimen. Also, when using the h-SAA3-3-1 antibody in particular as the human SAA3 antibody, specific binding to human SAA3 in the human specimen allows highly accurate detection and measurement.

As a method for detecting and measuring human SAA3 in a human specimen, any of the various methods employed in standard immunoassays such as the RIA method, the ELISA method, the fluorescent antibody method, or the agglutination method can be used. In these methods, the human SAA3 antibody of the invention can be bound to human SAA3 by means of the direct adsorption method, the sandwich method, or the competition method.

Typically, the method for detecting and measuring human SAA3 in a human specimen using the human SAA3 antibody of the invention is described below. First, human SAA3 polyclonal (polyclonal antibodies from a rabbit or the like, antibodies other than those of a mouse) antibodies produced according to standard methods are immobilized in the solid phase on a plate or a microparticle and then the human specimen is allowed to react with the solid phase human SAA3 antibodies. After washing, the human SAA3 antibody is allowed to react as a secondary antibody with the solid phase human SAA3. The secondary antibody may be directly labeled as HRP, ALP, RI or the like and used, or it may be used unlabeled and thereafter a tertiary antibody such as an anti-mouse IgG is measured as being labeled with HRP, ALP, RI or the like. The appropriate measurement method can be selected from among the RI method, the colorimetric method, the luminescence method, the gold colloid method and the like based on the respective sensitivities.

It is also possible, for the purpose of enabling detection and measurement, to allow the human SAA3 antibody of the invention to be labeled or immobilized in the solid phase onto a plate or a microparticle to provide a human SAA3 detection and measurement kit.

Typically, the human SAA3 detection and measurement kit will comprise an antibody solid phase plate or microparticle, washing solutions, the human SAA3 antibodies of the invention, anti-mouse IgG labeled antibodies, a color developing agent and a reaction terminating agent.

By utilizing these human SAA3 detection and measurement methods and kits, the concentration of human SAA3 in a human specimen can be detected and measured at a sensitivity of 0.1 to 10 ng/mL.

In addition, since human SAA3 expression is unique to cancer patients, particularly those experiencing metastasis to the brain, liver, lung or the like, the cancer or cancer metastasis can be diagnosed by detecting and measuring human SAA3 in a human specimen using the human SAA3 detection and measurement methods and kits.

Typically, cancer is diagnosed on the basis of the detection of human SAA3 in a human specimen such as serum, urine or the like from a healthy individual (including cancer patients who have not been diagnosed as having cancer). Cancer metastasis is also diagnosed on the basis of detecting human SAA3 in a human specimen such as serum, urine or the like from cancer patients. Concentration of human SAA3 in cancer patients with metastasis to the lung was substantially higher than those with metastasis to sites other than the lung. Accordingly, human SAA3 concentration in cancer patients is monitored routinely using the aforementioned human SAA3 detection and measurement methods and kits. When this concentration is elevated a diagnosis can be made of metastasis to the lung.

It is also known that the mouse SAA3 antibody inhibits the metastasis of cancer (Non-Patent Document 1). Thus, the human SAA3 antibody of the invention, when administered to cancer patients and thereby affecting the binding of this antibody with blood, serum, tissue or the like of in vivo human SAA3 , can also inhibit the effect of human SAA3 on TLR4, thus being useful as a therapeutic agent for cancer by inhibiting cancer infiltration including metastasis, as well as in therapeutic treatments for cancer. As used herein, cancer infiltration including metastasis refers to the expansion of the cancer into surrounding tissues (metastasis), and the successive advancement of a previously identified metastasis. Also, since the concentration of human SAA3 in cancer patients with metastasis to the lung is substantially higher than that of cancer patients with metastasis to sites other than the lung as described above, the human SAA3 antibody of the invention can inhibit cancer metastasis especially to the lung.

When the h-SAA3-3-1 antibody in particular is used as the human SAA3 antibody of the invention and is employed as the therapeutic agent or therapeutic method for cancer as described above, specific binding to human SAA3 which is in the human specimen or present in vivo can provide an efficient therapeutic agent or therapeutic method for cancer.

When using the human SAA3 antibody as a therapeutic agent for human cancer by inhibiting cancer infiltration including metastasis, antibodies obtained from an animal other than humans are used, preferably after being modified into chimeric antibodies, humanized antibodies, fully human antibodies or antibody fragments thereof using gene engineering methodology.

As used herein, a chimeric antibody is an animal antibody in which the constant region is replaced with the constant region of a human antibody. Such a chimeric antibody can be produced based on the method described, for example, in the document by Co et al (Co MS and Queen C, Humanized antibodies for therapy, Nature 351.501(1991)).

As used herein, a humanized antibody is an animal antibody in which the human SAA3 binding region is replaced with the binding region of a human antibody. Such a humanized antibody can be produced based on the method described, for example, in the document by Riechmann et al (L. Riechmann, M. Clark, H. Waldmann, G. Winter, Reshaping human antibodies for therapy. Nature 332. 323(1998)).

Also as used herein, a fully human antibody is an antibody similar to that produced by humans. Such a fully human antibody can be produced based on the method described, for example, in the document by Green et al (Green LL, Hardy MC, Maynard-Currie CE et al, Antigen-specific human monoclonal antibodies from mice engineered with human Ig heavy and light chain YACs, Nature Genetics 7. 13(1994)).

Also as used herein, an antibody fragment is an antibody which has been fragmented by an enzyme or the like, and includes those in which the human SAA3 binding region such as Fv, Fab or F(ab')₂ and the like is preserved.

Chimeric antibodies, humanized antibodies, fully human antibodies and their antibody fragments can also be obtained from various organizations through tailored services.

The respective antibodies described above may further be purified as desired and then formulated according to standard methods. The dosage type of this therapeutic cancer agent can be a parenteral type such as an injection formulation. This therapeutic cancer agent is given preferably via intravenous administration. Known carriers and additives suitable for the dosage type can be employed in converting the agent into the desired formulation.

When using the human SAA3 antibodies of the invention as therapeutic cancer agent as described above, the daily dose of the human SAA3 antibody can be 5 to 100 mg/individual, and preferably 2 to 10 mg/individual, although this may vary depending on the type of cancer, and the age, stature, gender and other factors of the relevant cancer patient, as well as the nature of the antibody.

### Examples

The present invention is further described in the following examples, but these are not intended to restrict the present invention in any way.

### Example 1

### Preparation of human SAA3 monoclonal antibody:

A synthetic peptide of positions 38 to 60 of human SAA3 consisting of the amino acid sequence represented by SEQ ID NO:1 (obtained from Operon Biotechnologies) (hereinafter this peptide is referred to as 23-SAA3) was conjugated with ovalbumin (OVA) as the carrier protein using EMCS (maleimide method) to obtain an OVA synthetic peptide.

Two mg of this OVA synthetic peptide was dissolved in 0.3 ml of physiological saline and then combined with 0.6 ml of 50% polyvinylpyrrolidone (PVP) and stirred, and then combined with 1.1 ml of complete Freund's adjuvant, and subjected to an OmniMixer (50,000 rpm) to yield an ointment-like adjuvant (A. Arimura, H. Sato, T. Kumasaka, et al., Production of Antiserum to LH-RH-Releasing Hormone (LH-RH) Associated with Gonadal Atrophy in Rabbits: Development of Radio-immunoassays for LH-RH, Endocrinology. 93 1092(1973)).

Each mouse (3 animals) was given 0.2 ml of the adjuvant obtained above via subcutaneously administration. Administration was conducted in the first, third and fifth week and after these three dosings, a small amount of blood was taken from the tail of each mouse and examined for the antibody titer. The mouse exhibiting an increased antibody titer was immunized again (booster) and its spleen was extracted 3 days after this immunization. The extracted splenocytes were fused with myeloma cells (P₃X63Ag8·U1(P₃U1)) using polyethylene glycol. Thereafter, the cells were distributed into 200 ml of HAT medium, 100 µl of which was dispensed to each of ten 96-well microplates. On the 10th and 15th days after the cell fusion, an assay was conducted using a 23-SAA3 solid phase-based ELISA method.

As a result, 15 cells were markedly 23-SAA3 positive. Each of these hybridoma cells was transferred to a 24-well plate where the 2nd assay was conducted. Finally, 5 cells exhibiting potent antibody titers were selected.

Subsequently, these five hybridoma cells were recloned in a methyl cellulose medium to obtain the monoclonal antibody hybridoma cells.

The five hybridoma cells obtained above were cultured in RPMI1640 medium supplemented with 10%FCS to allow 5 human SAA3 monoclonal antibodies to be produced. Using a solid phase (microplate 10ng/well to which 23-SAA3, a synthetic peptide of positions 49 to 60 of human SAA3 consisting of the amino acid sequence represented by SEQ ID NO:1 (obtained from Operon Biotechnologies) (hereinafter this peptide is referred to as 12-SAA3) and a synthetic peptide of a mouse SAA1 (hereinafter this peptide is referred to as a "synthetic peptide") were added, the reactivities of the five human SAA3 monoclonal antibodies were investigated. The solid phase was blocked using a TBS-T (containing 10% HS) solution, and the secondary antibody was diluted with a TBS-T(IgG+IgM) solution. As a positive control, mouse serum was employed after being subjected to a 50-fold dilution. The measurement device employed was a MultiScan JX manufactured by Thermo Fisher Scientific KK. The results are shown in Table 1. The subclasses of these antibodies were determined using a mouse monoclonal isotyping test kit (HyCult biotechnology). The results are shown in Table 2.

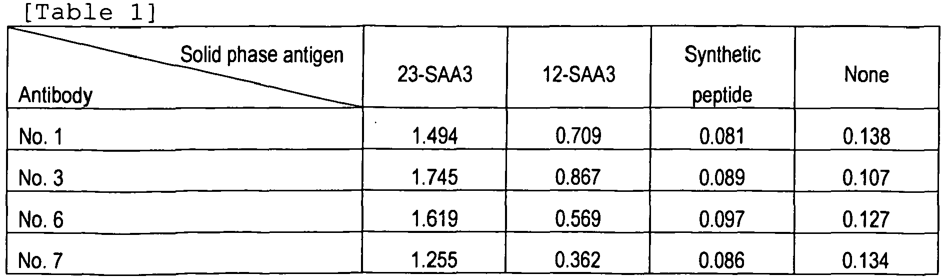

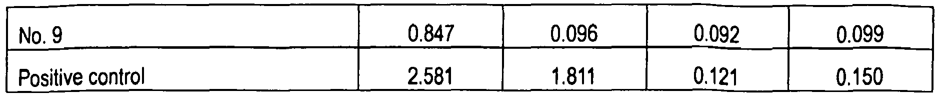

**[Table 2]**

| | No.1 | No.3 | No.6 | No.7 | No.9 |
|---|---|---|---|---|---|
| Subclass | IgG1 | IgG1 | IgG1,A | IgG2b | IgG2a |

Based on these results, antibodies No.1, 3, 6 and 7 were revealed to be monoclonal antibodies which specifically recognize the 12-SAA3 in the N-terminal region of the human SAA3. On the other hand, antibody No.9 did not recognize 12-SAA3 but did recognize 23-SAA3, and accordingly was revealed to be a monoclonal antibody which specifically recognizes positions 38 to 48 of human SAA3 consisting of the amino acid sequence represented by SEQ ID NO:1. It was also revealed that the subclass of all the antibodies was IgG.

### Comparative Example 1

### Preparation of human SAA3 monoclonal antibody:

A synthetic peptide of positions 49 to 60 of human SAA3 consisting of the amino acid sequence represented by SEQ ID NO:1 (obtained from Operon Biotechnologies) (hereinafter this peptide is referred to as 12-SAA3) was conjugated with ovalbumin (OVA) as a carrier protein using EMCS (maleimide method) to obtain an OVA synthetic peptide. Two mg of this OVA synthetic peptide was dissolved in 0.3 ml of physiological saline and then combined with 0.6 ml of 50% polyvinylpyrrolidone (PVP) and stirred, and then combined with 1.1 ml of complete Freund's adjuvant, and subjected to an OmniMixer (50,000 rpm) to yield an ointment-like adjuvant. Each mouse (3 animals)was given 0.2 ml of the adjuvant obtained above via subcutaneous administration to. The administration was conducted at 2 to 3 week intervals over a period of 6 to 8 months, but only a slight increase in the antibody titer was observed. No effect was observed also when the adjuvant was replaced with several others such as TiterMax Gold (CytRx Corporation). A prolonged immune sensitization resulted in fibrogenesis in the spleen observed upon the cell fusion, with a few satisfactory cells and no positive hybridomas obtained.

### Comparative Example 2

### Human serum amyloid-A3 expression using E.coli:

The human serum amyloid-A3 gene sequence (Accession Number: AY209188) was cloned and integrated into a vector pGEX4T-1 and then allowed to be expressed in E.coli. This E.coli was cultured in an LB medium. When human SAA3 was purified from this E.coli, the amount of protein obtained was as little as 1 mg. Although the procedure described in Comparative 1 was employed to produce the adjuvant which was then given twice, no antibody was produced and the experiment was discontinued due to a low level of efficiency.

### Example 2

### Detection of human SAA3 in human serum:

Antibody No. 9 obtained in Example 1, a human SAA3 monoclonal antibody (h-SAA3-3-1 antibody), and the samples listed below were employed to conduct a western blot analysis according to standard methodology. The detection in the western blot analysis was conducted using the ECL PLUS luminescence method. The results are shown in Figure 1.

### <Samples>

Serum from healthy individual: B3-2, 4-2, Y8-2
Serum from cancer patient: 151-2^{*1}, 167-2^{*2}, 169-2^{*3}

^{*}1: Gastric cancer patient with brain metastasis upon serum sampling
^{*}2: Large intestine cancer patient with liver metastasis upon serum sampling
^{*}3: Gastric cancer patient with lung metastasis upon serum sampling Expression proteins (positive controls) : SAA1, SAA2, SAA3^{*4}, SAA4
^{*}4: Obtained in Comparative example 2

The results of the western blot analysis confirmed the presence of human SAA3 in three samples of cancer patients. The concentration of human SAA3 in the sample from the gastric cancer patient with lung metastasis (169-2) was significantly high. Thus, human SAA3 appeared to be expressed at a high concentration in the cancer patient with lung metastasis while it appeared to be expressed at a low concentration in the cases of brain or liver metastasis, indicating that human SAA3 concentration is involved in lung metastasis.

### Example 3

### Human SAA3 detection and measurement kit:

The monoclonal antibodies which were antibodies No.1, 3, 6 and 7 obtained in Example 1 were immobilized in the solid phase in the wells of a microplate, which was then blocked and washed. Also, the h-SAA3-3-1 antibody obtained in Example 1 was subjected to a Fab treatment, and then labeled with horseradish peroxidase (HRP). The microplate and the HRP-labeled antibody were combined with an HRP color development substrate 3,3',5,5'-tetramethylbenzidine (TMB) to produce a human SAA3 detection and measurement kit.

Recombinant SAA3 or serum (human specimen) was added to the well of the microplate of the human SAA3 detection and measurement kit produced above and allowed to react for 1 hour at room temperature. After washing the well, 4µg/mL of HRP-labeled h-SAA3-3-1 antibody was added and allowed to react for 1 hour at room temperature. After washing the well, TMB was added and an enzymatic reaction was conducted for 30 minutes at room temperature, after which the reaction was terminated and the Abs 450 was measured.

### Industrial Applicability

Since the human SAA3 antibody of the invention binds to human SAA3, it can be utilized in studying what role human SAA3 plays in vivo in the human body.

Also, since mouse SAA3 is implicated in cancer, the human SAA3 antibody of the invention can be utilized in the treatment and diagnosis of cancer.

## Claims

1. A human serum amyloid-A3 antibody to counter human serum amyloid-A3.

2. The human serum amyloid-A3 antibody according to Claim 1 which is a monoclonal antibody.

3. The human serum amyloid-A3 antibody according to Claim 1, which is obtained by:
binding a peptide comprising positions 49 to 60 of the amino acid sequence of a human serum amyloid-A3 represented by SEQ ID NO:1 as a partial amino acid sequence with a biopolymeric compound to form a conjugate;
immunizing an animal with the conjugate and
obtaining an antibody from the animal.

4. The human serum amyloid-A3 antibody according to Claim 3 wherein the peptide to be bound with the biopolymer is the amino acid sequence of positions 38 to 60 of the amino acid sequence represented by SEQ ID NO:1

5. The human serum amyloid-A3 antibody according to Claim 1, which recognizes the peptide consisting of positions 49 to 60 of the amino acid sequence of the human serum amyloid-A3 represented by SEQ ID NO:1.

6. The human serum amyloid-A3 antibody according to Claim 1, which recognizes the peptide consisting of positions 38 to 48 of the amino acid sequence of the human serum amyloid-A3 represented by SEQ ID NO:1.

7. The human serum amyloid-A3 antibody according to Claim 1 which is a chimeric antibody.

8. The human serum amyloid-A3 antibody according to Claim 1 which is a humanized antibody.

9. The human serum amyloid-A3 antibody according to Claim 1 which is a fully humanized antibody.

10. The human serum amyloid-A3 antibody according to Claim 1 which is an antibody fragment.

11. A method for producing a human serum amyloid-A3 antibody comprising:
binding a peptide comprising positions 48 to 60 of the amino acid sequence of a human serum amyloid-A3 represented by SEQ ID NO:1 as a partial amino acid sequence with a biopolymeric compound to form a conjugate; and
immunizing an animal with the conjugate and obtaining antibodies from the animal.

12. The method for producing a human serum amyloid-A3 antibody according to Claim 11 wherein the peptide to be bound with the biopolymeric compound is the amino acid sequence of positions 38 to 60 of the amino acid sequence represented by SEQ ID NO:1.

13. A method for detecting and measuring a human serum amyloid-A3 comprising allowing the human serum amyloid-A3 antibody to act on a human specimen to detect and measure a human serum amyloid-A3 in the specimen.

14. A method for diagnosing a cancer comprising allowing the human serum amyloid-A3 antibody to act on a human specimen to detect and measure a human serum amyloid-A3 in the specimen.

15. The method for diagnosing a cancer according to Claim 14 wherein cancer infiltration including metastasis is diagnosed.

16. A detection and measurement kit for a human serum amyloid-A3 comprising use of a human serum amyloid-A3 antibody.

17. The detection and measurement kit for a human serum amyloid-A3 according to Claim 16 for cancer diagnosis.

18. The detection and measurement kit for a human serum amyloid-A3 according to Claim 16 for diagnosing cancer infiltration including metastasis.

19. A therapeutic cancer agent comprising a human serum amyloid-A3 antibody as an active ingredient.

20. The therapeutic cancer agent according to Claim 19 which inhibits cancer infiltration including metastasis.

21. A method for treating a cancer comprising administering a human serum amyloid-A3 antibody to a cancer patient and allowing the antibody to bind with in vivo human serum amyloid-A3.

22. The method for treating a cancer according to Claim 21 wherein cancer infiltration including metastasis is inhibited.


SEQUENCE LISTING

<110>  Tokyo Women's Medical University

<120>  An antibody for hSAA3 and use thereof

<130>  PF-100022-WO

<150>  JP2009-230361
<151>  2009-10-02

<160>  1

<170>  PatentIn version 3.5

<210>  1
<211>  60
<212>  PRT
<213>  Homo sapiens

<220>
<221>  MISC_FEATURE
<223>  Inventor:Yoshiro, MARU; Takeshi, TANAKA; Kiyomi, TERAI

<220>
<221>  SIGNAL
<222>  (1)..(18)

<220>
<221>  mat_peptide
<222>  (19)..(60)

<400>  1

Met Lys Leu Ser Thr Gly Ile Ile Phe Cys Ser Leu Val Leu Gly Val
            -15                 -10                 -5

Ser Ser Gln Gly Trp Leu Thr Phe Leu Lys Ala Ala Gly Gln Gly Thr
    -1  1               5                   10

Lys Asp Met Trp Lys Ala Tyr Ser Asp Met Lys Glu Ala Asn Tyr Lys
15                  20                  25                  30

Lys Phe Arg Gln Ile Leu Pro Cys Leu Gly Glu Leu

35 40